# FASCICULE DE BREVET EUROPEEN

(11) **EP 3 880 060 B1**
(45) Date de publication et mention de la délivrance du brevet: **31.12.2025**
(21) Numéro de dépôt: 19801805.3
(22) Date de dépôt: 06.11.2019
(51) Int. Cl.: A61B 5/00

(54) **SYSTÈME D'IMAGERIE 2D ET 3D D'UN DÉSORDRE PIGMENTAIRE CUTANÉ**
2D- UND 3D-ABBILDUNGSSYSTEM FÜR EINE HAUTPIGMENTSTÖRUNG
2D AND 3D IMAGING SYSTEM FOR A CUTANEOUS PIGMENT DISORDER

(30) Priorité: 15.11.2018 FR 1871843
(43) Date de publication de la demande: 22.09.2021
(73) Titulaire: THALES, 92190 Meudon (FR); Sispia, 94300 Vincennes (FR)
(72) Inventeur: BERECHET, Ion, 94300 Vincennes (FR); BERGINC, Gérard, 78995 Elancourt (FR); BERECHET, Stefan, 94340 Joinville le Pont (FR)
(74) Mandataire: Atout PI Laplace
(86) Numéro de dépôt international: PCT/EP2019/080330
(87) Numéro de publication internationale: WO 2020/099198

(56) Documents cités:
- WO-A1-2009/115947
- US-A1- 2011 213 253
- GAËL RIGAUD ET AL: "Reflective tomography solved by the inverse Radon transform", 1 October 2015 (2015-10-01), XP055611561, Retrieved from the Internet <URL:https://pdfs.semanticscholar.org/01be/bb5a91f9c4bf463bdebce40a0652c5155417.pdf> [retrieved on 20190807]
- TIMOTHY J. MULDOON ET AL: "Analysis of skin lesions using laminar optical tomography", BIOMEDICAL OPTICS EXPRESS, 22 June 2012 (2012-06-22), pages 1701 - 1712, XP055311125, Retrieved from the Internet <URL:https://www.ncbi.nlm.nih.gov/pmc/articles/PMC3395492/pdf/1701.pdf> [retrieved on 20161017], DOI: 10.1364/BOE.3.001701

## Description

Le domaine de l'invention est celui de l'imagerie des désordres pigmentaires de la peau.

La dermatologie fait face à une demande croissante de prise en charge de plus en plus précoce des pathologies cutanées. Dans la problématique des désordres pigmentaires de la peau, les statistiques de l'Organisation Mondiale de Santé (OMS) montrent :
- 2 à 3 millions de carcinomes, 132 000 cas de mélanomes dans le monde en 2011,
- un doublement du nombre des cas tous les 10 ans depuis 1945,
- dans 15 à 20 % des cas, le mélanome se développe à partir d'un grain de beauté,
- 15% de chance de survie à 5 ans si la détection est tardive,
- 95% de chance de guérison si la détection est précoce.

Pour diagnostiquer les désordres pigmentaires de la peau (grains de beauté, lésions bénignes, lésions malignes, carcinomes, mélanomes ...), les techniques dermatologiques sont basées sur des critères appellés ABCDE. Ainsi, l'évolution d'un désordre pigmentaire vers un cancer se caractérise par :
- une asymétrie (A) du mélanome,
- une bordure (B) irrégulière,
- une couleur (C) inhabituelle,
- un diamètre (D) qui évolue
- une évolution (E) inhabituelle.

De plus, les dermatologues essaient d'évaluer la non-uniformité de l'épaisseur du désordre pigmentaire à l'aide des techniques d'imagerie bidimensionnelle du type dermoscopie ou dermatoscopie non appropriées à une caractérisation fiable de l'évolution en profondeur du désordre pigmentaire. Dans certains cas pour confirmer le diagnostic, la pratique couteuse de biopsies et d'examens d'histopathologie est réalisée.

Le défi des dermatologues est d'identifier les « grains de beauté » suspects le plus précocement possible et de développer une nouvelle sémiologie, c'est-à-dire la correspondance entre ce que l'on observe et la pathologie.

Dans le cadre du diagnostic des désordres pigmentaires, la technique la plus répandue, l'épiluminescence ou dermatoscopie, repose sur l'utilisation d'un vidéo microscope le plus souvent numérique afin que l'image soit analysée à l'aide des fameux critères ABCD. Plus récemment, de nouveaux équipements sont apparus, comme par exemple le Siascope de la société Astron Clinica et le MelaFind de la société Electro-Optical Systems. Ils sont basés tous les deux sur une information multispectrale dans le visible et le proche infrarouge et se différencient par les outils de traitement de l'information associés.

Le Siascope est associé à un modèle physique simple et offre à l'utilisateur des images calculées de la mélanine et de l'hémoglobine. Dans une version plus évoluée, le praticien dispose d'une information sur la localisation en profondeur de la mélanine, information très importante pour le diagnostic de certaines lésions pigmentaires. Cependant la localisation en profondeur est liée à la précision du modèle mathématique utilisé. Un modèle complet de rétrodiffusion de la lumière sur la peau et sa structure interne reste très difficile à obtenir, très peu de données sont disponibles dans la littérature pour valider cette approche.

Le système MelaFind, comme son nom l'indique, analyse les images de taches pigmentaires dans le but unique de la détection du mélanome. Les images de ces taches pigmentaires sont des ortho images. Les outils numériques associés ne reposent pas sur un modèle physique, mais consistent essentiellement en un algorithme de classification qui compare toute nouvelle tâche à la base de données existante et retourne à l'opérateur une réponse de type oui/non. Ce type de dispositif peut correspondre aux enjeux du diagnostic, voire du dépistage ou de la prévention, mais pas aux enjeux de l'action thérapeutique et du suivi.

Dans la pratique, on utilise en complément une biopsie cutanée avec histologie. Mais cette approche est peu à même de remonter à des paramètres biochimiques du sujet. Elle reste dominée par l'indice de Breslow, mesurant l'épaisseur maximum de la tumeur sur coupe histologique, et est donc limitée par le nombre et la qualité des coupes, minorée artificiellement par les aspects régressifs avec des marges standardisées consensuelles mais empiriques dépendant de l'épaisseur de la tumeur appréciée par Breslow ; les marges ne sont pas toujours compatibles avec la topographie de la tumeur (visage), elles sont standardisées et dépendent de la taille cliniquement visible de la tumeur (jusqu'à 1 cm) et sont difficiles à préciser dans certaines formes cliniques (carcinome basocellulaire sclérodermiforme et micronodulaire).

Il existe bien d'autres pathologies cutanées d'origines diverses mais souvent liées aux principaux chromophores de la peau, avec des appareils dédiés à une seule pathologie (cas du MelaFind, comme nous l'avons décrit précédemment).

Le document WO2009/115947A1 divulgue un dispositif d'imagerie de la peau, comprenant un boitier équipé d'une fenêtre de visualisation, au moins un moyen d'éclairage pour éclairer à l'intérieur du boitier, au moins un moyen d'acquisition d'image en champ proche, et au moins un moyen d'acquisition d'image en champ lointain. L'acquisition de l'image est effectuée en bistatique.

Le but de l'invention est de pallier ces inconvénients.

Plus précisément l'invention a pour objet un système d'imagerie d'un désordre pigmentaire cutané qui comporte :
- un dispositif d'acquisition d'images 2D du désordre pigmentaire comprenant :
   ▪ une source lumineuse configurée pour éclairer ledit désordre comportant au moins un émetteur et
   ▪ des récepteurs,
- une unité de traitement des images acquises,
- des moyens de visualisation des images traitées.

Il est principalement caractérisé en ce qu'il comporte en outre :
- des moyens de positionnement des récepteurs selon au moins deux angles de vue de manière à obtenir au moins une image par angle de vue, et répartis selon une calotte sphérique,
- une structure de protection du dispositif d'acquisition et d'un opérateur, comportant une fenêtre de positionnement des récepteurs de manière à les diriger vers le désordre pigmentaire, la structure étant destinée à être positionnée sur la peau et présentant une surface externe opacifiée sauf sur la fenêtre de positionnement,
- en ce que l'unité de traitement comporte un module de reconstruction 3D à transformation de Radon réflective de manière à obtenir une image 3D reconstruite à partir des images 2D traitées,
- et en ce que les moyens de visualisation comportent en outre des moyens de visualisation de l'image 3D reconstruite.

Les dimensions de la structure de protection sont avantageusement compatibles d'un dispositif d'acquisition portable.

Selon une caractéristique de l'invention, le dispositif d'acquisition comprend un pavé multicellulaire avec un récepteur par cellule.

Selon une autre caractéristique de l'invention, le dispositif d'acquisition comporte une source lumineuse émettant dans la bande visible, et des moyens d'acquisition des images simultanément par les récepteurs.

Les moyens de positionnement comportent par exemple un rail sur lequel est (sont) positionné(s) un (ou plusieurs) récepteur(s), et des moyens de rotation du rail. Le rail peut être coulissant avec un seul récepteur ou un seul émetteur-récepteur.

La source lumineuse peut être multi longueur d'onde dans les bandes visible et proche infra-rouge, et/ou la bande de la 1ére fenêtre thérapeutique et/ou la bande de la 2ème fenêtre thérapeutique et/ou la bande SWIR, le (ou les) récepteur(s) correspondant auxdites longueurs d'onde et étant synchronisé(s) avec les émetteurs, et le dispositif d'acquisition comprenant des moyens d'acquisition des images successivement par les récepteurs à raison d'au moins une image par longueur d'onde et par angle de vue.

Grâce notamment à une illumination de la peau dans les bandes visible et proche infrarouge (0,4 µm à 1.1 µm) et/ou SWIR (Small Wave InfraRed, de 1,1 µm à 2,2 µm), et/ou celles des 1^{ère} ou 2éme fenêtre thérapeutiques, ce nouveau système d'imagerie permet la visualisation 2D à divers angles de vue, la visualisation volumique 3D des désordres pigmentaires et de leurs racines cutanées, en utilisant un dispositif optique non-invasif et des modules de traitement et de visualisation 2D et 3D associés.

Le système qui repose sur une illumination de volume, permet une observation directe après reconstruction tridimensionnelle du désordre pigmentaire dans sa profondeur.

Le système selon l'invention est suffisamment générique pour s'adapter si ce n'est à toutes, tout du moins à un grand nombre de pathologies cutanées. Du fait de la visualisation 3D volumique qui permet une vision externe mais aussi interne du désordre pigmentaire, il permet de révéler des structures et des signes invisibles à l'œil nu (à l'intérieur), d'améliorer la performance du diagnostic clinique des lésions pigmentaires, de détecter des paramètres permettant d'affiner le diagnostic différentiel entre mélanomes et nævus (localisation, quantité ou qualité de la mélanine, mise en évidence de phénomènes de néovascularisation autour du mélanome, profondeur de la structure du mélanome...).

Il est possible grâce à l'imagerie volumique 3D combinée avec l'imagerie bidimensionnelle 2D à divers angles de vue de mieux visualiser les limites tridimensionnelles du désordre pigmentaire et de l'éventuelle tumeur, d'être certain de l'exérèse complète de la tumeur cutanée maligne, des conditions de la guérison et d'améliorer le pronostic fonctionnel.

D'autres caractéristiques et avantages de l'invention apparaîtront à la lecture de la description détaillée qui suit, faite à titre d'exemple non limitatif et en référence aux dessins annexés dans lesquels :
la figure 1 est une illustration d'un désordre pigmentaire, par exemple un nævus,
la figure 2 représente schématiquement un exemple de système d'imagerie selon l'invention,
la figure 3a représente une coupe verticale d'un dispositif d'acquisition d'images 2D à divers angles en version coupole,
la figure 3b représente une vue en perspective d'un dispositif d'acquisition d'images 2D à divers angles en version coupole,
la figure 3c représente une coupe horizontale d'un dispositif d'acquisition d'images 2D à divers angles en version coupole,
la figure 4 représente schématiquement les éléments fonctionnels d'un dispositif d'acquisition d'images 2D,
la figure 5a représente une coupe verticale d'un dispositif d'acquisition d'images 2D à divers angles en version coupole avec jupe,
la figure 5b représente une vue en perspective d'un dispositif d'acquisition d'images 2D à divers angles en version coupole avec jupe,
la figure 6a représente une coupe verticale d'un dispositif d'acquisition d'images 2D à divers angles en version coupole multicellulaire avec jupe,
la figure 6b représente une vue en perspective d'un dispositif d'acquisition d'images 2D à divers angles en version coupole multicellulaire avec jupe,
la figure 7 est une illustration d'images 2D d'un nævus acquises selon divers angles et post-traitées,
les figures 8 forment un ensemble d'illustrations de reconstruction tridimensionnelle par rendu de voxels du volume 3D complet d'un nævus et de zooms de la structure tridimensionnelle du nævus considéré, avec pour la figure 8a une image de la surface du nævus selon un angle de vue, pour la figure 8b une image de la surface du nævus selon un autre angle de vue, pour la figure 8c une image en profondeur du nævus selon un angle de vue, pour la figure 8d une image en profondeur du nævus selon un autre angle de vue.

D'une figure à l'autre, les mêmes éléments sont repérés par les mêmes références.

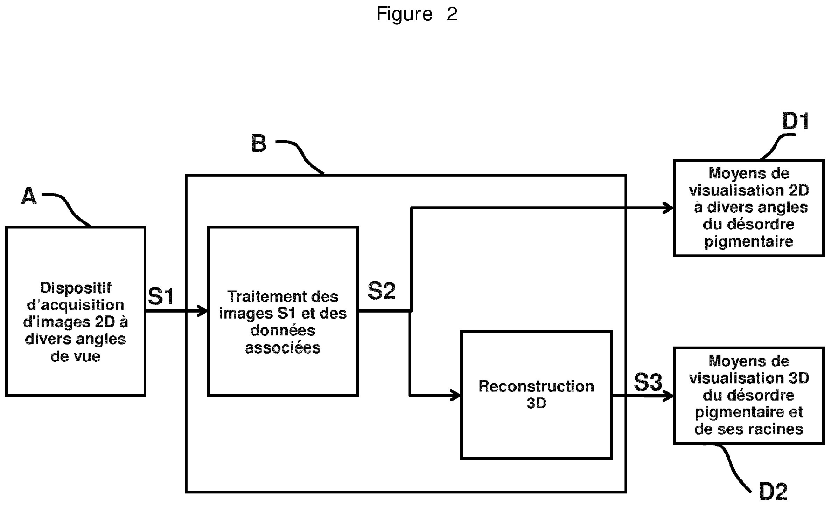

Selon l'invention, le système d'imagerie des désordres pigmentaires décrit en relation avec la figure 2 est destiné à être utilisé par un opérateur qui peut être un professionnel de santé mais pas nécessairement. Le système d'imagerie comporte les éléments suivants :
❖ Un dispositif A d'acquisition d'images 2D à divers angles de vue et éventuellement à diverses longueurs d'onde pour chaque angle de vue, qui génère des images 2D brutes S1 et les données associées (angles de vue et longueur d'onde).
❖ Une unité B de traitement d'images qui génère des images 2D traitées S2 à partir des images brutes S1 et des données associées et qui de préférence génère aussi une reconstruction 3D S3 du désordre pigmentaire et de ses racines à partir des images traitées S2.
❖ Des moyens D1 de visualisation 2D à divers angles de vue du désordre pigmentaire à partir des images 2D traitées S2.
❖ Des moyens D2 de visualisation 3D du désordre pigmentaire et de ses racines reconstruites S3.

L'unité B de traitement d'images et/ou les moyens D1 et D2 de visualisation peuvent être déportés du dispositif d'acquisition A.

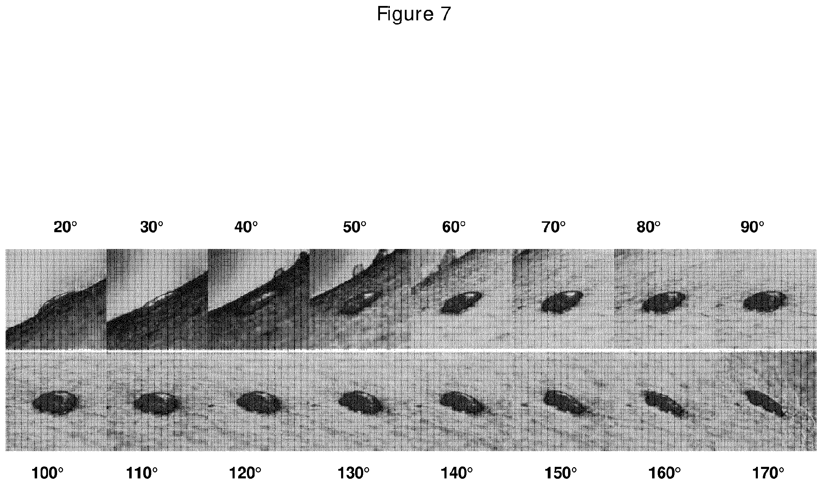

L'unité B de traitement d'images réalise les conversions RAW vers un format image exploitable (JPEG, PNG, TIFF, ...), les corrections des aberrations optiques, le cadrage, le centrage, le recalage, le calibrage, la mise en échelle, le seuillage, et l'indexation angulaire des images brutes S1. On obtient des images traitées S2. Des exemples d'images 2D traitées S2 d'un nævus à divers angles de prise de vue (20°, 30°, ..., 160°, 170°) sont donnés figure 7.

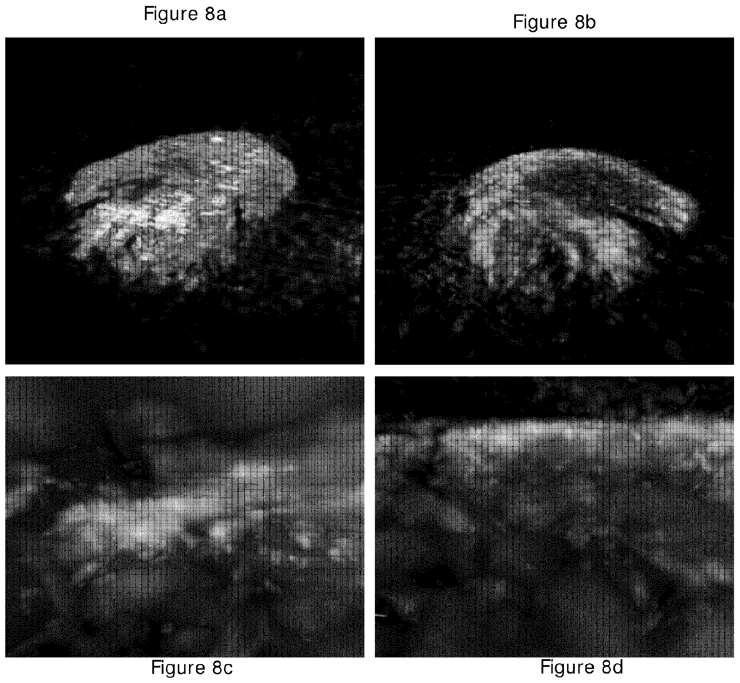

De préférence comme montré figure 2, à partir des images traitées S2 (elles-mêmes issues des images S1 représentant les niveaux d'intensité d'un rayonnement électromagnétique réfléchi ou émis par la surface de l'objet), l'unité B assure aussi la reconstruction tridimensionnelle du désordre pigmentaire via un processus algorithmique de type tomographie réflective fondée sur la transformation inverse de Radon. On obtient des images traitées S3. Concernant cette reconstruction tridimensionnelle, on peut par exemple se reporter au brevet US 8,836,762 « ,Optronic system and method dedicated to identification for formulating three-dimensional Images ». Ce type de reconstruction utilise des données liées à la réflexion, à la diffusion ou à l'émission électromagnétique de l'onde lumineuse incidente sur les structures du désordre pigmentaire (nævus, carcinome, mélanome...) dans sa profondeur ; elle permet une reconstruction tridimensionnelle totale (de type tomographique) du désordre pigmentaire comme illustré figure 8, sans introduire de paramètres d'absorption des tissus.

Ces images S3 sont de type nuage de points, isodensités, rendu de voxels (par exemple Maximum Intensity Projection (MIP), comme décrit dans le brevet EP 3 234 914 B1 : procédé de discrimination et d'identification par imagerie 3D d'objets d'une scène. La technique MIP permet de visualiser des données tridimensionnelles dans un plan bidimensionnel. Les voxels (Volume Pixels) sont projetés sur un plan 2D ; les voxels sont déterminés par les rayons joignant le plan de projection au point d'observation et en appliquant aux voxels un seuil d'intensité imposé. Plusieurs plans de projection sont créés à des angles d'observation successifs pour obtenir une notion de profondeur et ainsi améliorer le rendu 3D.Une illustration d'un rendu des voxels du volume 3D reconstruit (S3) d'un nævus est donnée dans les figures 8 : les figures 8a et 8b montrent une image de la surface d'un naevus, prise selon deux angles de vue, les figures 8c et 8d montrent une image en profondeur du naevus prise selon deux angles de vue.

Les moyens D1 et D2 de visualisation 2D (des images S2) et 3D (des images S3) sont typiquement des PC, des tablettes, des téléphones portables (« smartphones ») ou tout autre moyen de visualisation.

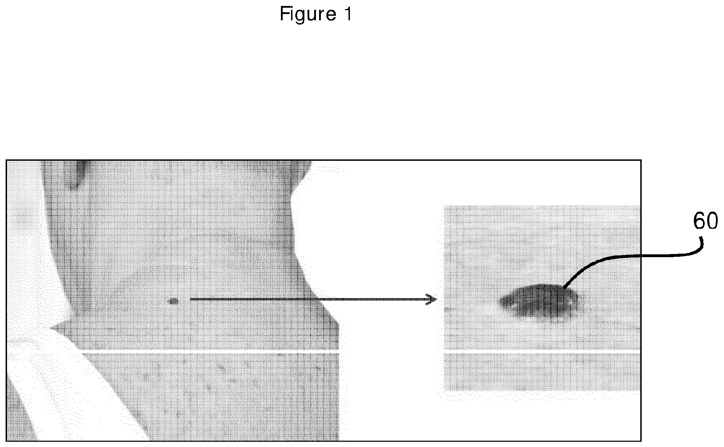

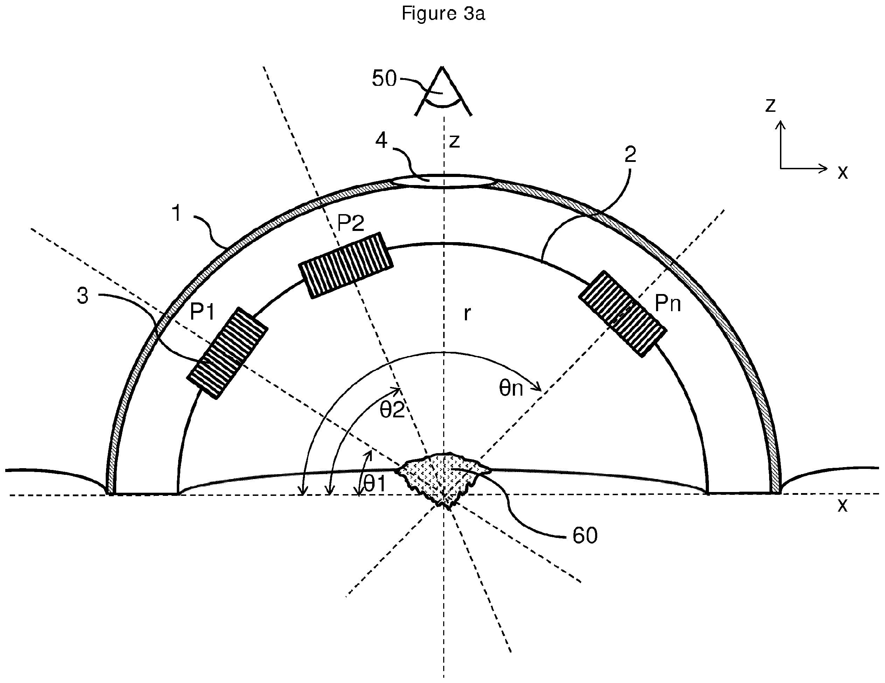

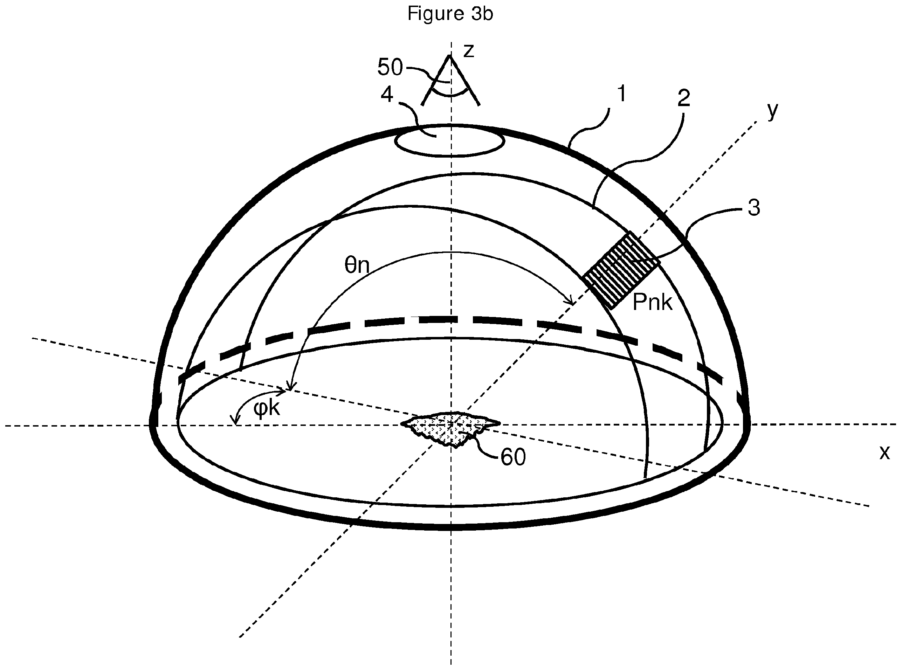

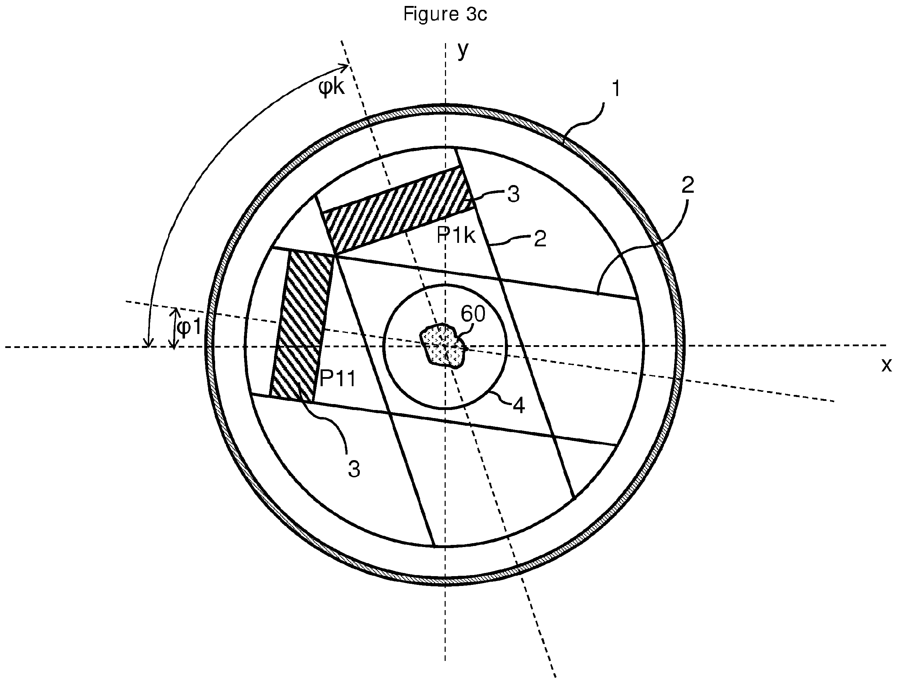

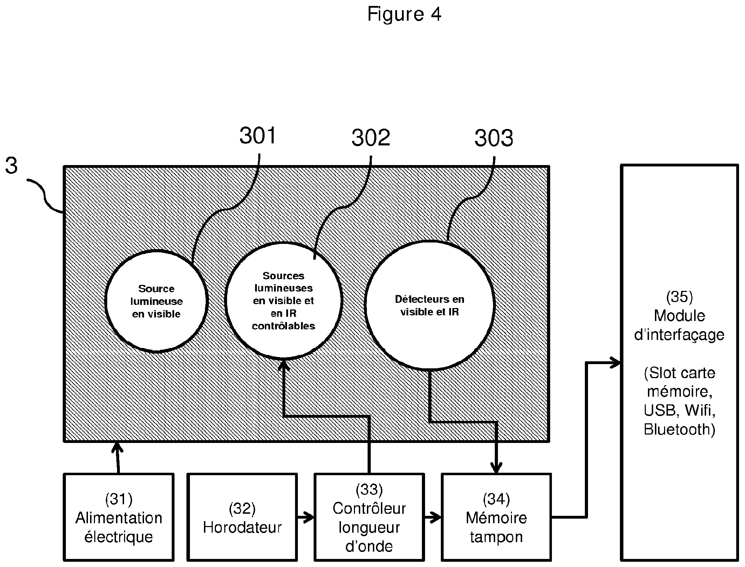

On va à présent décrire plus en détail le dispositif A d'acquisition d'images 2D à divers angles de vue du désordre pigmentaire, montré figure 3a, 3b et 3c. Il comporte :
- selon un mode d'exécution qui ne fait pas partie de l'invention, une source lumineuse configurée pour éclairer le désordre pigmentaire. Elle comporte un ou plusieurs émetteurs orientés vers le désordre pigmentaire 60 (dont un exemple est montré figure 1). La source lumineuse peut être une source visible qui émet dans la bande 0,6 µm-0,8 µm. De préférence la source lumineuse est mono longueur d'onde et contrôlable en longueur d'onde et elle émet successivement dans les bandes visible et proche infrarouge (0,4 µm-1,1 µm), les bandes des 1ére (0,65 µm-0,95 µm) et/ou 2eme (1 µm-1,35 µm) fenêtres thérapeutiques et/ou la bande SWIR (1,1 µm-2,2 µm), avec une longueur d'onde par bande.
- un ou plusieurs récepteurs 303 (montrés figure 4) adaptés aux longueurs d'onde du ou des émetteurs. En sortie des récepteurs, on obtient les images S1.

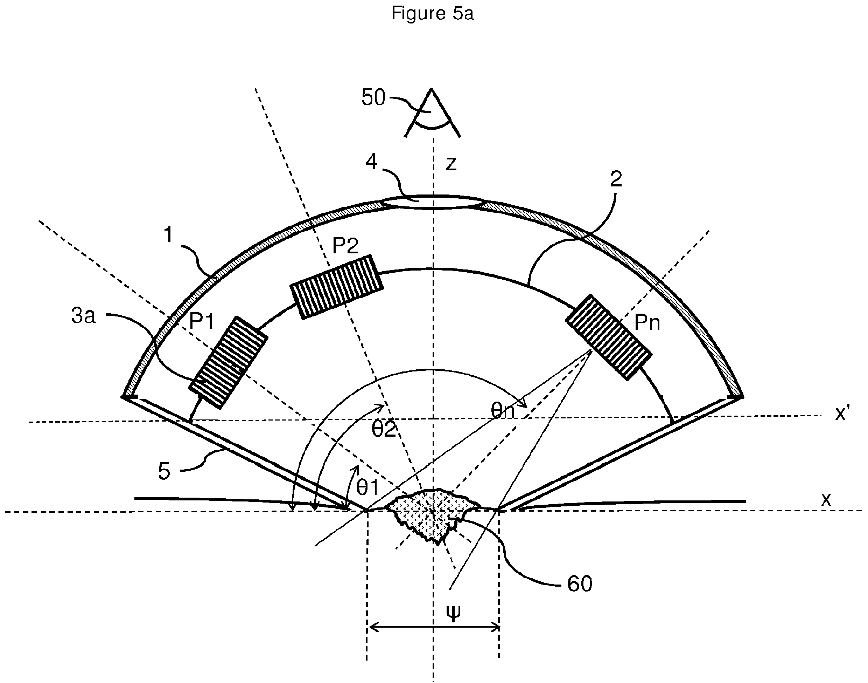

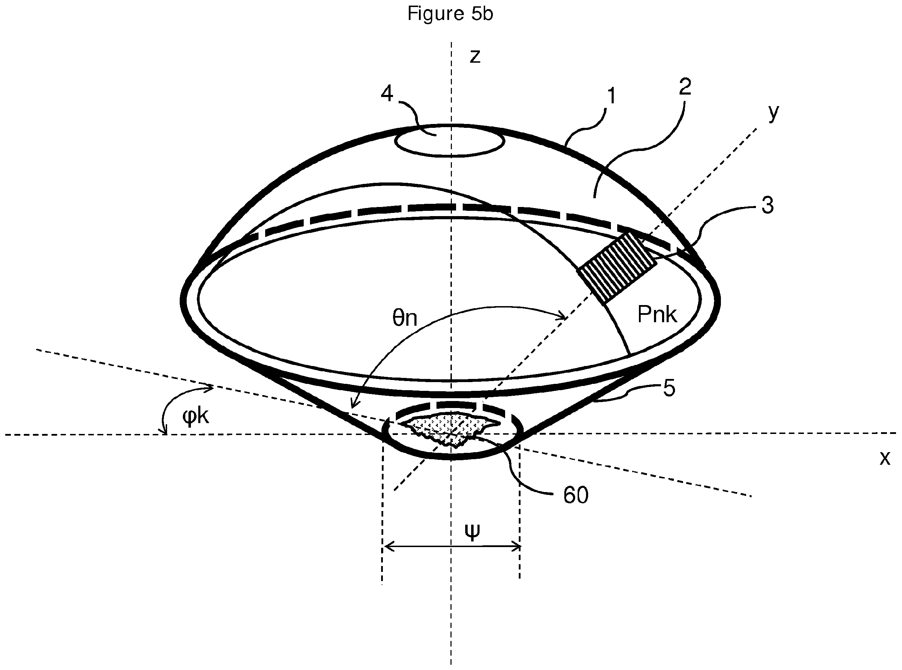

Selon un mode d'exécution de l'invention, on peut avoir des dispositifs désignés émetteurs-récepteurs 3 intégrant la fonction émission et la fonction réception dans le même dispositif comme montré sur les figures 3a, 3b, 3c, 5a, 5b, 6a. Un exemple d'émetteur-récepteur 3 est détaillé figure 4. Il comporte une source lumineuse 301 dans la bande visible, et aussi de préférence des sources lumineuses 302 dans les bandes visible et IR, contrôlables, reliées à un contrôleur en longueur d'onde 33 lui-même relié à un horodateur 32. Le contrôleur en longueur d'onde 33 et l'horodateur 32 peuvent être hébergés par l'unité de traitement B. Le dispositif 3 comporte aussi des récepteurs dans les bandes correspondant à celles des sources lumineuses (visible et IR).
- des moyens de positionnement du ou des récepteurs (voire aussi du ou des émetteurs) selon M angles de vue (M≥2) du désordre pigmentaire et répartis selon une calotte sphérique comme on peut le voir figures 3a, 3b, 5a et 5b, de manière à obtenir au moins une image par angle de vue θn (n varie de 1 à N, avec N≤M), les images étant acquises à la même distance du désordre pigmentaire. Plus θN - θ1 est grand, plus les images visualisées 2D et/ou 3D seront précises. On a typiquement 120° ≤ θN - θ1 ≤ 180° ; il y a 16 angles de vue sur la figure 7 avec θ1= 20°, θN =170° et θN - θ1 = 150°. Les moyens de positionnement comportent par exemple un rail 2 arrondi selon cette calotte sphérique, sur lequel peut coulisser un récepteur (voire un émetteur-récepteur 3) à des positions Pn assurant un angle de vue θn. On peut aussi utiliser un rail 2 sur lequel des récepteurs sont fixés à des positions Pn déterminées a priori (il y a donc N récepteurs), sans qu'il soit nécessaire de les faire coulisser. Les moyens de positionnement comportent également des moyens de rotation du rail 2 autour de son axe vertical Oz (comme montré figure 3c sur laquelle on peut voir deux positions du rail 2) à des angles φk (k varie de 1 à K, K≤M et N.K=M) assurant des positions Pnk du ou des récepteurs (voire des émetteurs-récepteurs 3) comme montré figure 3b et 3c ou autour d'un axe horizontal Ox situé dans un plan contenant les extrémités du rail. Plus φK - φ1 est grand, plus les images visualisées 2D et/ou 3D seront précises. On a typiquement 120° ≤ φK - φ1 ≤ 180°. Selon une alternative, les moyens de positionnement sont solidaires de la structure de protection 1 décrite ci-dessous plus en détail et c'est la structure 1 qui tourne manuellement ou automatiquement. On notera que θN - θ1 = φK - φ1=180° correspond à une structure en forme de coupole comme montré sur les figures 3a et 3b.

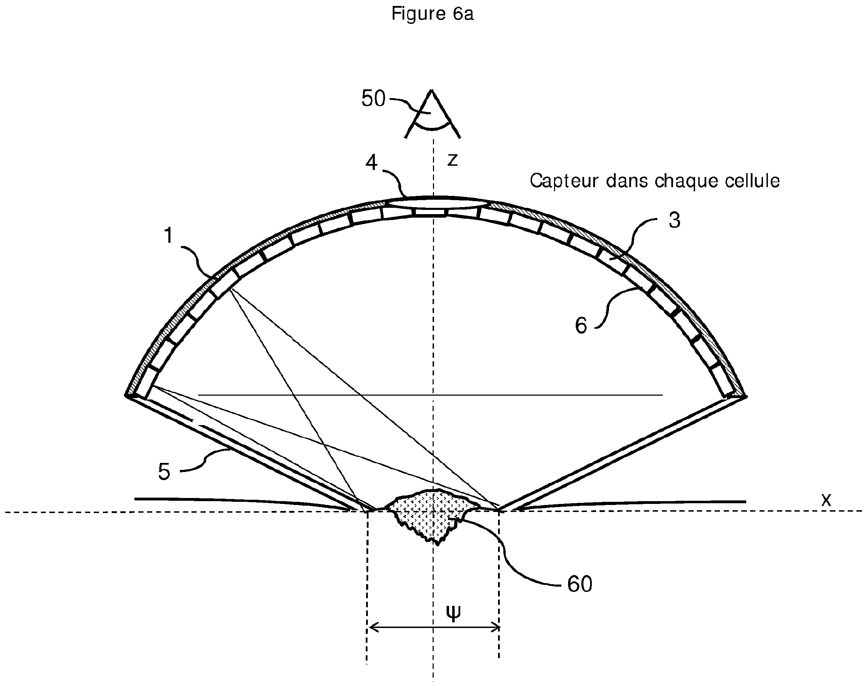

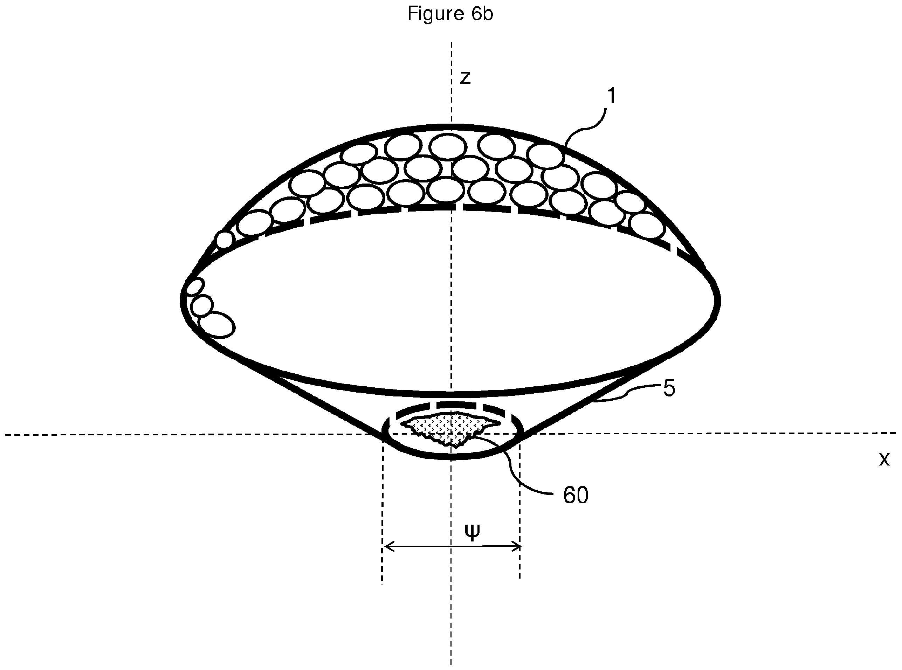

Selon un mode de réalisation, le dispositif d'acquisition comprend un pavé multicellulaire, chaque cellule 6 contenant un récepteur, voire un émetteur-récepteur 3. Ce pavé a la forme de la calotte sphérique comme montré figure 6a et 6b ; il peut être distribué sur une zone limitée ou sur toute la surface intérieure de la calotte.

Le dispositif d'acquisition peut être adapté ou intégré à un téléphone portable (smartphone).
- le dispositif d'acquisition comprend en outre une structure de protection 1 de l'opérateur 50 et des émetteurs et récepteurs, qui généralement épouse la forme des moyens de positionnement comme montré sur les figures, mais d'autres formes peuvent être envisagées (cubique, ou composée ou autre). Cette structure 1 comporte une fenêtre de positionnement du dispositif d'acquisition de manière à le diriger vers le désordre 60. La fenêtre de positionnement est disposée au sommet de la structure 1 de protection, à la normale du désordre 60 selon l'axe z (la peau est disposée dans un plan xy). Ainsi, l'opérateur peut positionner, par inspection visuelle directe, le dispositif d'acquisition, afin que la fenêtre soit face au désordre 60, comme illustré sur les figures 3a et 3b, tout en maintenant la structure 1 de protection sur la peau. Cette fenêtre peut comporter une loupe 4 facilitant un centrage visuel sur le désordre pigmentaire 60 par l'opérateur 50. La structure 1 est destinée à être positionnée sur la peau par l'opérateur et présente une surface externe opacifiée sauf sur la fenêtre de positionnement de manière à protéger l'opérateur des émissions lumineuses et aussi pour protéger les récepteurs d'émissions parasites. Dans un souci de sécurité oculaire, la loupe 4 comporte avantageusement un obturateur automatique lors de l'émission-réception.

On a représenté sur les figures 3a et 3b une structure 1 en forme de coupole. Selon une variante montrée figure 5a et 5b, une jupe 5 est associée à la structure de protection 1 pour permettre l'appui sur la peau et des ouvertures Ψ adaptables aux dimensions du désordre pigmentaire 60 et/ou à sa position sur telle ou telle partie du corps, comme le visage par exemple.

De préférence les dimensions de la structure 1 sont compatibles d'un dispositif d'acquisition A portable facile à poser sur différentes parties du corps : lorsque la structure 1 forme par exemple une coupole comme montré sur les figures, celle-ci a typiquement un rayon < 15cm. La structure peut aussi être fixe et de dimensions plus grandes.

A titre d'exemple non-limitatif, l'interfaçage (35) est de type : slot carte mémoire, USB, Wifi, Bluetooth, ....

Deux techniques d'acquisition d'images peuvent être utilisées :
- Selon un mode d'execution qui ne fait pas partie de l'invention, technique d'imagerie « passive »: elle concerne les bandes visible et proche infrarouge. Le désordre pigmentaire est éclairé par la lumière ambiante extérieure au dispositif passant à travers la fenêtre de positionnement éventuellement équipée de la loupe 4, qui reste transparente à la lumière ambiante. L'émetteur considéré est alors celui de la lumière ambiante. Les images 2D sont acquises successivement pour le dispositif décrit en relation avec les figures 5a et 5b. Les images peuvent être acquises simultanément par l'ensemble du pavé de récepteurs multicellulaires pour le dispositif d'acquisition décrit en relation avec les figures 6a et 6b. Le dispositif d'acquisition comprend alors des moyens d'acquisition simultanée, tels que des moyens de synchronisation des récepteurs. L'éclairement ambiant permet d'obtenir une image 3D de la surface volumique du désordre mais ne permet pas toujours d'obtenir une profondeur importante pour l'image tridimensionnelle reconstruite.
- Selon un mode d'exécution de la présente invention, technique d'imagerie « active » : elle concerne les bandes visible et infrarouge. Le désordre pigmentaire est éclairé par les sources émettrices 301, 302 des émetteurs-récepteurs 3, la fenêtre de positionnement n'étant plus transparente à la lumière ambiante extérieure au dispositif ; cette fenêtre est par exemple masquée par l'opérateur ou peut aussi être équipée d'un obturateur comme indiqué précédemment. L'éclairement peut être réalisé successivement dans plusieurs longueurs d'ondes pour chaque angle de vue. Le désordre pigmentaire est alors imagé successivement pour chaque longueur d'onde et pour chaque angle de vue. Le dispositif d'acquisition comprend alors des moyens d'acquisition successive synchronisés avec les éclairements, tels que des moyens de synchronisation des récepteurs avec les émetteurs. Cette technique permet d'obtenir une image 3D de la surface volumique du désordre et une imagerie tridimensionnelle profonde du désordre pigmentaire. Avantageusement, à chaque prise de vue, les angles d'émission et de réception sont orientés et égaux ou quasi-égaux par rapport à la normale au plan contenant la peau. Ces angles (θ, φ) sont illustrés sur les figures 3a, 3b et 3c dans le repère (x,y,z). On définit ainsi un repère angulaire par rapport à la normale, où deux angles de part et d'autre de la normale peuvent avoir la même valeur absolue mais sont de signes contraires. Ainsi, les images bidimensionnelles sont collectées dans une configuration monostatique ou quasi-monostatique. Le dispositif est ainsi conçu pour utiliser des images bidimensionnelles en rétroflexion ou réflexion monostatique ou quasi-monostatique afin d'obtenir une image tridimensionnelle à partir de l'ensemble des images bidimensionnelles prises à différents angles. En outre, l'imagerie 3D sera d'autant plus précise que le nombre d'images bidimensionnelles collectées et précisément référencées sera important.

L'invention permet d'obtenir une image volumique tridimensionnelle par illumination dans la « 1ère fenêtre thérapeutique » (650 nm - 950 nm) ; cette approche conduit à une résolution spatiale accrue et à un bruit de fond minimisé. L'utilisation de l'imagerie tridimensionnelle, dont les propriétés spectroscopiques (absorption photonique et émission) se situent dans la première fenêtre thérapeutique, permet l'accès à l'imagerie de tissus dit « épais ».

La « seconde fenêtre thérapeutique » (1000 nm - 1350 nm), permet d'augmenter la profondeur de pénétration de l'onde du fait de la minimisation de diffusion des photons, et on peut obtenir ainsi une transmission lumineuse sur plusieurs millimètres de profondeur. Cette fenêtre d'émission apporte des informations supplémentaires sur l'évolution des racines du nævus et sa micro-vascularisation.

La diffusion de l'onde lumineuse émanant des différentes longueurs d'onde utilisées permet un ensemble de reconstructions tridimensionnelles des désordres pigmentaires et donc une information complète et comparative des désordres pigmentaires sachant que les phénomènes de diffusion des ondes lumineuses sur les désordres pigmentaires sont complémentaires (profondeur de diffusion, section efficaces de diffusion, détermination des rugosités de la structure diffusante) suivant les longueurs d'ondes d'éclairement utilisées.

Ainsi, les images bidimensionnelles obtenues sont utilisées pour obtenir une reconstruction tridimensionnelle dans la profondeur de la peau notamment pour les longueurs d'onde d'éclairement possédant un fort pouvoir de pénétration des tissus de la peau.

Ce système d'imagerie 2D et 3D s'applique principalement au domaine biomédical dans l'identification de désordres cutanés ou sous-cutanés. Dans le cadre des cancers cutanés non mélaniques (carcinome basocellulaire, carcinome spinocellulaire par exemple), le système selon l'invention permet une visualisation précise des limites tridimensionnelles de la tumeur ce qui permet d'être certain de l'exérèse complète de la tumeur cutanée maligne qui est une condition de la guérison.

Ainsi, les formes nodulaires simples les plus fréquentes (45 à 60 pour 100 des cas) sont très bien limitées, mais on peut avoir au sein de ces formes simples, une forme micronodulaire, sans délimitation périphérique et nécessitant des marges d'exérèse plus importantes, l'image tridimensionnelle permettant une représentation précise de ces formes complexes.

Ainsi l'imagerie tridimensionnelle permet de révéler des structures et des signes invisibles à l'œil nu, améliorant la performance du diagnostic clinique des lésions pigmentées, de détecter des paramètres tridimensionnels permettant d'affiner le diagnostic différentiel entre mélanomes, carcinomes et nævus (localisation, forme, profondeur, mise en évidence de phénomènes de vascularisation autour du mélanome/carcinome, modifications du microrelief, modifications de la jonction dermoépidermique, modifications vasculaires, visualisation des limites de la tumeur ce qui permet d'être certain de l'exérèse complète de la tumeur cutanée).

## Revendications

1. Système d'imagerie d'un désordre pigmentaire cutané (60) qui comporte :
- un dispositif (A) d'acquisition d'images 2D du désordre pigmentaire dans une configuration monostatique ou quasi-monostatique, comprenant au moins un émetteur-récepteur (3), l'émetteur-récepteur comprenant au moins une source lumineuse (301, 302) configurée pour éclairer ledit désordre, et au moins un récepteur (303) dont la longueur d'onde correspond à celle de l'émetteur
- une unité (B) de traitement des images 2D acquises (S1), pour obtenir des images 2D traitées (S2),
- des moyens (D1) de visualisation des images traitées (S2),
**caractérisé en ce qu'**il comporte en outre :
- des moyens de positionnement l'émetteur-récepteur (3) selon au moins deux angles de vue de manière à obtenir au moins une image par angle de vue, et répartis selon une calotte sphérique,
- une structure (1) de protection du dispositif d'acquisition (A) et d'un opérateur (50), comportant une fenêtre de positionnement de l'émetteur-récepteur (3) de manière à le diriger vers le désordre pigmentaire (60), la structure de protection étant destinée à être positionnée sur la peau et présentant une surface externe opacifiée,
- **en ce que** l'unité (B) de traitement comporte un module de reconstruction 3D à transformation de Radon réflective de manière à obtenir une image 3D reconstruite (S3) à partir des images 2D traitées (S2),
- et **en ce que** les moyens de visualisation comportent en outre des moyens de visualisation (D2) de l'image 3D reconstruite (S3).

2. Système d'imagerie d'un désordre pigmentaire cutané selon la revendication précédente, **caractérisé en ce que** la structure (1) de protection présente des dimensions compatibles d'un dispositif d'acquisition (A) portable.

3. Système d'imagerie d'un désordre pigmentaire cutané selon l'une des revendications précédentes, **caractérisé en ce que** le dispositif d'acquisition (A) comprend un pavé multicellulaire, chaque cellule (6) contenant un émetteur-récepteur (3).

4. Système d'imagerie d'un désordre pigmentaire cutané selon la revendication précédente, **caractérisé en ce que**, à chaque prise de vue, l'angle d'émission et l'angle de réception sont orientés et égaux par rapport à la normale au plan contenant la peau.

5. Système d'imagerie d'un désordre pigmentaire cutané selon l'une des revendications 3 ou 4, **caractérisé en ce que** le dispositif d'acquisition comporte une source lumineuse émettant dans la bande visible et des moyens d'acquisition des images (S1) simultanément par les récepteurs.

6. Système d'imagerie d'un désordre pigmentaire cutané selon la revendication 1 à 3, **caractérisé en ce que** les moyens de positionnement comportent un rail (2) sur lequel est (sont) positionné(s) un (ou plusieurs) émetteur-récepteur(s) et des moyens de rotation du rail.

7. Système d'imagerie d'un désordre pigmentaire cutané selon la revendication précédente, **caractérisé en ce que** le dispositif d'acquisition comprend un seul émetteur-récepteur (3) sur le rail (2) et **en ce que** le rail est coulissant.

8. Système d'imagerie d'un désordre pigmentaire cutané selon l'une des revendications 1, 2, 3, 4 ou 7, **caractérisé en ce que** la source lumineuse est multi longueur d'onde dans les bandes visible et proche infra-rouge, et/ou la bande de la 1ére fenêtre thérapeutique et/ou la bande de la 2ème fenêtre thérapeutique et/ou la bande SWIR, le (ou les) récepteur(s) correspondant auxdites longueurs d'onde et étant synchronisé(s) avec les émetteurs, et **en ce que** le dispositif d'acquisition comprend des moyens d'acquisition des images (S1) successivement par les récepteurs à raison d'au moins une image par longueur d'onde et par angle de vue.

9. Système d'imagerie d'un désordre pigmentaire cutané selon l'une des revendications précédentes, **caractérisé en ce que** la fenêtre de positionnement est disposée au sommet de la structure (1) de protection.

10. Système d'imagerie d'un désordre pigmentaire cutané selon l'une quelconque des revendications précédentes, **caractérisé en ce qu'**il est portatif.

11. Système d'imagerie d'un désordre pigmentaire cutané selon l'une quelconque des revendications précédentes, **caractérisé en ce que** le dispositif (A) d'acquisition est intégré à un téléphone portable.

## Patentansprüche

1. Abbildungssystem für eine Hautpigmentstörung (60), das umfasst:
- eine Vorrichtung (A) zur 2D-Bilderfassung der Pigmentstörung in einer monostatischen oder quasi-monostatischen Konfiguration, umfassend mindestens einen Sender-Empfänger (3), wobei der Sender-Empfänger mindestens eine Lichtquelle (301, 302) umfasst, die konfiguriert ist, um die Störung zu beleuchten, und mindestens einen Empfänger (303), dessen Wellenlänge jener des Senders entspricht
- eine Bearbeitungseinheit (B) der erfassten 2D-Bilder (S1), um bearbeitete 2D-Bilder (S2) zu erhalten,
- Visualisierungsmittel (D1) der bearbeiteten Bilder (S2),
**dadurch gekennzeichnet, dass** es weiter umfasst:
- Mittel zum Positionieren des Sender-Empfängers (3) gemäß mindestens zwei Betrachtungswinkeln, um mindestens ein Bild je Betrachtungswinkel zu erhalten, und gemäß einer Kugelhaube verteilt,
- eine Schutzstruktur (1) der Erfassungsvorrichtung (A) und eines Bedieners (50), die ein Fenster zum Positionieren des Senders-Empfängers (3) beinhaltet, um ihn auf die Pigmentstörung (60) zu richten, wobei die Schutzstruktur dazu bestimmt ist, auf der Haut positioniert zu werden, und eine getrübte Außenoberfläche aufweist,
- dadurch, dass die Bearbeitungseinheit (B) ein 3D-Rekonstruktionsmodul mit reflektierender Radon-Transformation umfasst, um ein rekonstruiertes 3D-Bild (S3) aus den bearbeiteten 2D-Bildern (S2) zu erhalten,
- und dadurch, dass die Visualisierungsmittel weiter Visualisierungsmittel (D2) des rekonstruierten 3D-Bildes (S3) beinhalten.

2. Abbildungssystem einer Hautpigmentstörung nach dem vorstehenden Anspruch, **dadurch gekennzeichnet, dass** die Schutzstruktur (1) mit einer tragbaren Erfassungsvorrichtung (A) kompatible Abmessungen aufweist.

3. Abbildungssystem einer Hautpigmentstörung nach einem der vorstehenden Ansprüche, **dadurch gekennzeichnet, dass** die Erfassungsvorrichtung (A) einen vielzelligen Block umfasst, wobei jede Zelle (6) einen Sender-Empfänger (3) enthält.

4. Abbildungssystem einer Hautpigmentstörung nach dem vorstehenden Anspruch, **dadurch gekennzeichnet, dass** der Sendewinkel und der Empfangswinkel bei jeder Bildaufnahme in Bezug auf die Normale zur Ebene, welche die Haut enthält, ausgerichtet und gleich sind.

5. Abbildungssystem einer Hautpigmentstörung nach einem der Ansprüche 3 oder 4, **dadurch gekennzeichnet, dass** die Erfassungsvorrichtung eine Lichtquelle, die im sichtbaren Band aussendet, und Mittel zum Erfassen der Bilder (S1) gleichzeitig durch die Empfänger umfasst.

6. Abbildungssystem einer Hautpigmentstörung nach Anspruch 1 bis 3, **dadurch gekennzeichnet, dass** die Positionierungsmittel eine Schiene (2) beinhalten, auf der ein (oder mehrere) Sender-Empfänger und Drehmittel der Schiene positioniert sind.

7. Abbildungssystem einer Hautpigmentstörung nach dem vorstehenden Anspruch, **dadurch gekennzeichnet, dass** die Erfassungsvorrichtung einen einzigen Sender-Empfänger (3) auf der Schiene (2) umfasst, und dadurch, dass die Schiene gleitend ist.

8. Abbildungssystem einer Hautpigmentstörung nach einem der Ansprüche 1, 2, 3, 4 oder 7, **dadurch gekennzeichnet, dass** die Lichtquelle mehrere Wellenlängen in den sichtbaren Bändern und nahe Infrarot, und/oder dem Band des 1. therapeutischen Fensters und/oder dem Band des 2. therapeutischen Fensters und/oder dem SWIR-Band aufweist, wobei der oder die Empfänger den Wellenlängen entspricht/entsprechen, und mit den Sendern synchronisiert ist/sind, und dadurch, dass die Erfassungsvorrichtung Erfassungsmittel der Bilder (S1) nacheinander durch die Empfänger im Umfang von mindestens einem Bild je Wellenlänge und Betrachtungswinkel umfasst.

9. Abbildungssystem einer Hautpigmentstörung nach einem der vorstehenden Ansprüche, **dadurch gekennzeichnet, dass** das Positionierungsfenster am Scheitelpunkt der Schutzstruktur (1) angeordnet ist.

10. Abbildungssystem einer Hautpigmentstörung nach einem der vorstehenden Ansprüche, **dadurch gekennzeichnet, dass** es tragbar ist.

11. Abbildungssystem einer Hautpigmentstörung nach einem der vorstehenden Ansprüche, **dadurch gekennzeichnet, dass** die Erfassungsvorrichtung (A) in ein Mobiltelefon integriert ist.

## Claims

1. System for imaging a cutaneous pigment disorder (60) which comprises:
- a device (A) for acquiring 2D images of the pigment disorder in a monostatic or quasi-monostatic configuration, comprising at least one transmitter/receiver (3), the transmitter/receiver comprising at least one light source (301, 302), which is configured to illuminate the disorder, and at least one receiver (303), the wavelength of which corresponds to that of the transmitter,
- a unit (B) for processing the 2D images acquired (S1) in order to obtain processed 2D images (S2),
- means (D1) for displaying the processed images (S2),
**characterized in that** it further comprises:
- means for positioning the transmitter/receiver (3) in accordance with at least two viewing angles in order to obtain at least one image per viewing angle, and in a state distributed along a spherical cap,
- a structure (1) for protecting the acquisition device (A) and an operator (50), comprising a window for positioning the transmitter/receiver (3) in order to direct it towards the pigment disorder (60), the protection structure being intended to be positioned on the skin and having an opacified outer surface,
- **in that** the processing unit (B) comprises a 3D reconstruction module with reflective Radon transform in order to obtain a reconstructed 3D image (S3) from the processed 2D images (S2),
- and **in that** the display means further comprise display means (D2) of the reconstructed 3D image (S3).

2. System for imaging a cutaneous pigment disorder according to the preceding claim, **characterized in that** the protection structure (1) has dimensions which are compatible with a portable acquisition device (A).

3. System for imaging a cutaneous pigment disorder according to either of the preceding claims, **characterized in that** the acquisition device (A) comprises a multicellular pad, each cell (6) containing a transmitter/receiver (3).

4. System for imaging a cutaneous pigment disorder according to the preceding claim, **characterized in that**, with each exposure, the transmission angle and the receiving angle are orientated and equal relative to the normal to the plane containing the skin.

5. System for imaging a cutaneous pigment disorder according to either claim 3 or claim 4, **characterized in that** the acquisition device comprises a light source transmitting in the visible range and means for simultaneously acquiring the images (S1) by the receivers.

6. System for imaging a cutaneous pigment disorder according to claims 1 to 3, **characterized in that** the positioning means comprise a rail (2) on which one (or more) transmitter/receiver(s) is/are positioned and means for rotating the rail.

7. System for imaging a cutaneous pigment disorder according to the preceding claim, **characterized in that** the acquisition device comprises a single transmitter/receiver (3) on the rail (2) and **in that** the rail slides.

8. System for imaging a cutaneous pigment disorder according to any one of claims 1, 2, 3, 4 or 7, **characterized in that** the light source is multi-wavelength in the visible and near-infrared ranges, and/or the range of the 1st therapeutic window and/or the range of the second therapeutic window and/or the SWIR range, the receivers(s) corresponding to the wavelengths and being synchronized with the transmitters, and **in that** the acquisition device comprises means for acquiring images (S1) successively via the receivers as a result of at least one image per wavelength and per viewing angle.

9. System for imaging a cutaneous pigment disorder according to any one of the preceding claims, **characterized in that** the positioning window is arranged at the tip of the protection structure (1).

10. System for imaging a cutaneous pigment disorder according to any one of the preceding claims, **characterized in that** it is portable.

11. System for imaging a cutaneous pigment disorder according to any one of the preceding claims, **characterized in that** the acquisition device (A) is integrated in a mobile telephone.
